Europäisches Patentamt

European Patent Office

Office européen des brevets

(11) Veröffentlichungsnummer: **0 279 387**
**B1**

(12) **EUROPÄISCHE PATENTSCHRIFT**

(45) Veröffentlichungstag der Patentschrift:
02.05.90

(21) Anmeldenummer: 88102126.5

(22) Anmeldetag: 13.02.88

(51) Int. Cl.⁴: **C07C 315/06**

(54) Verfahren zur Isolierung von 4,4'-Dichlordiphenylsulfon.

(30) Priorität: 17.02.87 DE 3704931

(43) Veröffentlichungstag der Anmeldung:
24.08.88 Patentblatt 88/34

(45) Bekanntmachung des Hinweises auf die Patenterteilung:
02.05.90 Patentblatt 90/18

(84) Benannte Vertragsstaaten:
BE CH DE FR GB IT LI

(56) Entgegenhaltungen:
DE-A- 2 267 813
DE-A- 2 555 376

(73) Patentinhaber: BASF Aktiengesellschaft,
Carl-Bosch-Strasse 38, D-6700 Ludwigshafen(DE)

(72) Erfinder: Schaefer, Gerhard, Dr., Schafrippel 2,
D-6900 Heidelberg(DE)
Erfinder: Eilingsfeld, Heinz, Dr., Pierstrasse 9 a,
D-6710 Frankenthal(DE)
Erfinder: Neumann, Peter, Dr.,
Franz-Schubert-Strasse 1, D-6908 Wiesloch(DE)
Erfinder: Stumpp, Michael, Dr., Bismarckstrasse 120 a,
D-6700 Ludwigshafen(DE)

## Beschreibung

4,4'-Dichlordiphenylsulfon ist ein wichtiges Zwischenprodukt, das hauptsächlich für die Herstellung aromatischer Polysulfone und zur Synthese von Bis(4-aminophenyl)-sulfon, das man sowohl für die Lepratherapie als auch zur Härtung von Epoxidharzen benötigt, verwendet wird. Voraussetzung für diese Einsatzgebiete ist eine hohe Reinheit des 4,4'-Dichlordiphenylsulfons.

Man kann 4,4'-Dichlordiphenylsulfon z.B. nach dem in der DE-PS 1 087 592 beschriebenen Verfahren durch Umsetzung von Chlorbenzol mit einer Mischung aus Schwefeltrioxid und Dimethylpyrosulfat herstellen. In der DE-AS 2 252 571 wird die Synthese aus Chlorbenzol und Chlorbenzolsufonsäure beschrieben. Sie erfordert Temperaturen von 220 bis 260°C, so daß bei überatmosphärischem Druck gearbeitet werden muß.

Eine weitere Möglichkeit zur Herstellung von 4,4'-Dichlordiphenylsulfon ist die z.B. in der DE-PS 2 704 972 beschriebene nach einer Friedel-Crafts-Reaktion ablaufende Umsetzung von 4-Chlorbenzolsulfonsäurechlorid mit Chlorbenzol, wobei man als Katalysator z.B. Eisen-III-chlorid verwendet. Die Umsetzung zum Sulfon wird in Chlorbenzol als Lösungsmittel bei ca. 140°C durchgeführt.

Bei den beschriebenen Verfahren erhält man stets Gemische isomerer Dichlordiphenylsulfone, so daß nach der üblichen Aufarbeitung ein zusätzlicher Reinigungsschritt zur Gewinnung von isomerenreinem 4,4'-Dichlordiphenylsulfon erforderlich ist.

Aufgabe der Erfindung war die Bereitstellung eines Verfahrens zur Isolierung von weitgehend isomerenreinem 4,4'-Dichlordiphenylsulfon aus Mischungen von 4,4'-Dichlordiphenylsulfon mit den isomeren 2,4'- und 3,4'-Dichlorverbindungen, wie sie z.B. bei den o.g. Verfahren entstehen.

Nach dem Verfahren der Erfindung, durch das diese Aufgabe gelöst wird, isoliert man 4,4'-Dichlordiphenylsulfon aus einem Dichloridphenylsulfone enthaltenden Gemisch, das, bezogen auf 4,4'-Dichlordiphenylsulfon, 0,5 bis 20 Gew.% 2,4'-Dichlordiphenylsulfon und 0,5 bis 30 Gew.% 3,4'-Dichlordiphenylsulfon enthält, indem man dieses Gemisch bei Temperaturen von 20 bis 250°C mit einem Alkanol so behandelt, daß ein möglichst weitgehende Auflösung des Gemisches eintritt, aus dem so erhaltenen Gemisch durch Abkühlen reines 4,4'-Dichlordiphenylsulfon ausfällt, und das 4,4'-Dichlordiphenylsulfon in üblicher Weise abtrennt.

Gemische, aus denen man nach dem Verfahren dieser Erfindung weitgehend isomerenfreies 4,4'-Dichlordiphenylsulfon gewinnen kann, sind insbesondere Reaktionsgemische, die man nach an sich bekannten Verfahren zur Herstellung von 4,4'-Dichlordiphenylsulfon durch Umsetzung von Chlorbenzol mit sulfonierenden Verbindungen erhält. Solche Reaktionsgemische haben einen Gehalt an den isomeren Dichlordiphenylsulfonen von 10 bis 100, insbesondere 40 bis 100 Gew.% und können, je nach der Art ihrer Herstellung, unterschiedliche Mengen anderer Stoffe, wie Monomethylsulfat, Dimethylsulfat, Monethylsulfat, Diethylsulfat, Chlorbenzol, Eisenchlorid, Schwefelsäure, Chlorbenzolsulfonsäure und Chlorbenzolsulfonsäurechlorid enthalten.

Als Alkanole kommen beispielsweise $C_1$-$C_4$-Alkanole in Betracht. Die Verwendung von Methanol ist bevorzugt. Die Mengen an eingesetztem Alkanol kann in weiteren Grenzen variiert werden. Sie beträgt z.B. das 0,1 bis 10-fache, vorzugsweise das 0,5 bis 3-fache der Mengen an 4,4'-Dichlordiphenylsulfon im zu behandelnden Ausgangsgemisch.

Man behandelt das Ausgangsgemisch bei Temperaturen von 20 bis 250°C so mit dem Alkanol, daß eine möglichst weitgehende Auflösung des Gemisches eintritt. Durch Verminderung der Temperatur des Behandlungsgemisches wird dann das reine 4,4'-Dichlordiphenylsulfon ausgefällt und wie üblich abgetrennt, z.B. durch Filtration.

Im Einzelnen verfährt man z.B. so, daß man das aus einer der o.g. 4,4'-Dichlordiphenylsulfon-Synthesen stammende Reaktionsgemisch in vorgelegtes Methanol, gegebenenfalls unter Kühlung, einlaufen läßt. Entsteht aus dem Gemisch beim Sieden des Methanols keine Lösung, so arbeitet man zur Siedepunkterhöhung unter Druck. In der Regel sind Temperaturen von etwa 80 bis 160°C entsprechend Drücken von etwa 2 bis 18 bar ausreichend.

Bei Verwendung anderer Alkanole als Methanol kann es zur Vermeidung von Nebenreaktionen, wie Bildung von Olefinen, erforderlich sein, das Reaktionsgemisch vor dem Zulauf zum Alkohol abzukühlen. Bei Verwendung von Isobutanol entsteht bei ca. 100°C eine Lösung, aus der beim Abkühlen 4,4'-Dichlordiphenylsulfon mit einer Isomerenreinheit von ≧ 99,8 % ausfällt.

Nach den Angaben der DE-PS 1 087 592 wird das aus Chlorbenzol, Dimethylsulfat und Schwefeltrioxid erhältliche Reaktionsgemisch nach beendeter Umsetzung mit Wasser verdünnt. Bei dieser Operation fallen auch die nicht gewünschten Isomeren mit aus. Man erhält auf diese Weise lediglich ein Dichlordiphenylsulfon mit einer für Polymersynthesen ungenügenden Reinheit von ca. 95 % an 4,4'-Isomer. Ein weiteres Problem stellt die Entsorgung des in erheblicher Menge anfallenden Monomethylsulfats dar. Die Nachteile dieser Arbeitsweise werden durch das erfindungsgemäße Verfahren beseitigt: Man läßt das nach dem Verfahren der DE-PS 1 087 592 anfallende Reaktionsgemisch nicht in Wasser sondern in siedendes Methanol einlaufen. Durch Erhöhung der Temperatur auf ca. 105 bis 115°C unter Druck (3,5 bis 6 bar) geht das Dichlordiphenylsulfon-Gemisch in Lösung. Das nach dem Abkühlen ausfallende und durch Filtration isolierte 4,4'-Dichlordiphenylsulfon hat eine Isomerenreinheit von ≧ 99,8 % und ist nach dem Trocknen ohne weitere Reinigung für die Synthese von Polymeren einsetzbar.

Die erfindungsgemäße Arbeitsweise erlaubt auf einfache Weise die Rückführung des aus dem eingesetzten Dimethylsulfat stammenden Monomethylsulfats in den Reaktionskreislauf. Aus dem Mutterfiltrat wird das Alkanol (vorzugsweise Methanol) gegebenenfalls unter vermindertem Druck abdestilliert. Anschließend läßt sich aus dem alkanolfreien Rückstand durch Erhöhung der Temperatur auf 140 bis 150°C das Monomethylsulfat in an sich bekannter Weise in Dimethylsulfat überführen, das wieder für die Dichlordiphenylsulfonsynthese verwendet werden kann.

Auf ähnliche Weise läßt sich aus dem nach den Angaben der DE-AS 2 252 571 aus Chlorbenzol und 4-Chlorbenzolsulfonsäure erhältlichen Reaktionsgemisch reines 4,4'-Dichlordiphenylsulfon isolieren. Verwendet man dabei Methanol, so ist ein Abkühlen der über 200°C heißen Reaktionsmischung nicht erforderlich und man kann die Mischung mit Methanol bei Siedetemperatur des Methanols unter Normaldruck oder erhöhtem Druck durchführen. Bei Verwendung eines C2-C4-Alkanols ist es empfehlenswert, um die Wasserabspaltung zu Olefinen zu vermeiden, das Reaktionsgemisch vor der Eingabe in den Alkohol auf Temperaturen unter 100°C abzukühlen. Das nach dem Abkühlen und Ausfallen abgesaugte 4,4'-Dichlordiphenylsulfon hat eine Isomerenreinheit von ≧ 99,8 %. Nach Entfernung des Alkanols - falls erforderlich unter vermindertem Druck -kann man den Destillationssumpf in die Synthese zurückführen.

Man kann nach dem Verfahren der Erfindung auch die nach den Angaben der DE-PS 2 704 972 erhältlichen Reaktionsgemische besonders vorteilhaft aufarbeiten. Üblicherweise werden diese Gemische zur Entfernung des Einsenchlorids nach Zugabe an Chlorbenzol mit Wasser gewaschen. Erfindungsgemäß erhält man bei der unmittelbaren Behandlung der Reaktionsgemische mit den Alkanolen, gegebenenfalls nach Entfernung eines Großteils des als Lösungsmittel für die Synthese eingesetzten Chlorbenzols, eine auch den Katalysator FeCl₃ enthaltende Lösung. Nach dem Abkühlen und Absaugen wird 4,4'-Dichlordiphenylsulfon mit einer Reinheit von ≧ 99,8 % erhalten. Durch Rektifikation des Mutterfiltrats kann man Alkohol und Chlorbenzol zum Wiedereinsatz zurückgewinnen. Ein weiterer Vorteil des erfindungsgemäßen Verfahren ist somit die Tatsache, daß bei dieser Verfahrensweise der direkte Kontakt zwischen Reaktionsmischung und Wasser vermieden wird, so daß das erhaltene Mutterfiltrat vor oder nach Entfernung des Methanols ökotoxikologisch unbedenklich entsorgt werden kann.

Beispiel 1

Die Reaktionsmischung, die man nach den Angaben des Beispiels der DE-PS 1 087 592 durch Umsetzung von 160 g (2 mol) SO3 mit 225 g (2 mol) Chlorbenzol unter Zuhilfenahme von 126 g (1 mol) Dimethylsulfat erhält, wird mit einer Temperatur von 50°C in 200 g Methanol eingerührt. Das Gemisch hält man 1 h auf Siedetemperatur unter 3,5 bar Druck und kühlt dann auf 25°C ab. Das ausgefallene Produkt wird isoliert und 3 mal mit je 150 g Methanol gewaschen. Nach dem Trocknen erhält man 200 g eines Produktes, das bei 149 bis 150°C wasserklar schmilzt. Die Zusammensetzung (nach Gaschromatographie) ist:

≧ 99,8 % 4,4'-Dichlordiphenylsulfon
≦ 0,1 % 2,4'-Dichlordiphenylsulfon
≦ 0,1 % 3,4'-Dichlordiphenylsulfon

Aus den vereinigten Wasch- und Mutterlaugen wird das Methanol bis zu einer Sumpftemperatur von 100°C abdestilliert. Nach Beendigung der Methanoldestillation erhöht man die Innentemperatur auf 140 bis 150°C und destilliert unter Vakuum das entstehende Dimethylsulfat ab. Man gewinnt auf diesem Weg 90 g des eingesetzten Dimethylsulfats zurück.

Vergleichsbeispiel 1

Fällt man die Reaktionsmischung aus Beispiel 1 auf Wasser, so erhält man ein Produkt, da bei 138 bis 142°C mit brauner Farbe schmilzt und (nach Gaschromatographie) folgende Zusammensetzung hat:
95 % 4,4'-Dichlordiphenylsulfon
3,5 % 2,4'-Dichlordiphenylsulfon
1,5 % 3,4'-Dichlordiphenylsulfon

Beispiel 2

Man verfährt analog Beispiel 1, wobei man jedoch Methanol durch Isobutanol ersetzt. Die Mischung wird 1 Stunde bei 100°C gerührt und anschließend auf Raumtemperatur abgekühlt. Nach der Isolierung analog Beispiel 1 erhält man 190 g eines Produktes, das bei 150°C wasserklar schmilzt und folgende Zusammensetzung hat:
99,9 % 4,4'-Dichlordiphenylsulfon
0,1 % 2,4'-Dichlordiphenylsulfon

Beispiel 3

Die Reaktionsmischung, die nach dem Verfahren der DE-PS 27 04 972 durch Umsetzung von 250 g Chlorbenzolsulfochlorid, 200 ml Chlorbenzol und 10 g Eisen-III-chlorid bei 150 bis 160°C erhalten wurde, wird unter Siedekühlung in 450 ml Methanol eingerührt. Mann erhält die Suspension 2 Stunden unter Rückfluß, kühlt dann auf 40°C ab und isoliert das Produkt durch Filtration. Nach viermaligem Waschen mit je 300 ml Methanol und anschließendem Trocknen, erhält man 290 g Produkt mit einem Schmelzpunkt von 149°C und folgender Zusammensetzung (nach Gaschromatographie):
4,4'-Dichlordiphenylsulfon > 99,7 %
2,4'-Dichlordiphenylsulfon < 0,1 %
3,4'-Dichlordiphenylsulfon 0,1 %
Sonstige < 0,1 %

Durch Rektifikation gewinnt man 1500 ml Methanol und 60 ml Chlorbenzol zurück. Der verbleibende Destillationssumpf wird durch Verbrennung entsorgt.

Beispiel 4

Eine nach dem Verfahren der DE-PS 2 252 571 erhaltene 220°C heiße Reaktionsmischung aus 560 g isomeren Dichlordiphenylsulfonen, 210 g Chlorbenzolsulfonsäure und 10 g Chlorbenzol wird in 1000 ml Methanol eingerührt. Bei diesem Vorgang läßt man die Temperatur des Methanols auf 100°C ansteigen, so daß ein vollständige Auflösung des anfangs ausgefallenen Dichlordiphenylsulfons zu beobachten ist.

Nach dem Abkühlen, Abfiltrieren und intensivem Waschen mit Methanol erhält man 380 g Produkt mit einem Schmelzpunkt von 149 - 150°C und folgender Zusammensetzung:

4,4'-Dichlordiphenylsulfon 99,8 %
2,4'-Dichlordiphenylsulfon < 0,1 %
3,4'-Dichlordiphenylsulfon < 0,1 %
Sonstige < 0,1 %.

Der nach Destillation des Methanols zurückbleibende Sumpf besteht im wesentlichen aus isomeren Dichlordiphenylsulfonen und Chlorbenzolsulfonsäure und kann in den Reaktionskreislauf zurückgeführt werden.

## Patentansprüche

1. Verfahren zur Isolierung von 4,4'-Dichlordiphenylsulfon aus einem Dichlordiphenylsulfon enthaltenden Gemisch, das, bezogen auf 4,4'-Dichlordiphenylsulfon, 0,5 bis 20 Gew.% 2,4'-Dichlordiphenylsulfon und 0,5 bis 30 Gew.% 3,4'-Dichlordiphenylsulfon enthält, dadurch gekennzeichnet, daß man dieses Gemisch bei Temperaturen von 20 bis 250°C mit einem Alkanol so behandelt, daß ein möglichst weitgehende Auflösung des Gemisches eintritt, aus dem so erhaltenen Gemisch durch Abkühlen 4,4'-Dichlordiphenylsulfon ausfällt, und das 4,4'-Dichlordiphenylsulfon auf in üblicher Weise abtrennt.

2. Verfahren nach Anspruch 1, dadurch gekennzeichnet, daß man ein Alkanol mit 1 bis 4 C-Atomen verwendet.

3. Verfahren nach Anspruch 1. dadurch gekennzeichnet, daß man als Alkanol Methanol verwendet.

4. Verfahren nach Anspruch 1, dadurch gekennzeichnet, daß die Menge des eingesetzten Alkanols das 0,1 bis 10-fache der Gewichtsmenge an 4,4'-Dichlordiphenylsulfon im Ausgangsgemisch beträgt.

5. Verfahren nach Anspruch 1, dadurch gekennzeichnet, daß man solche Dichlordiphenylsulfongemische behandelt, die man nach an sich bekannten Verfahren zur Herstellung von 4,4'-Dichlordiphenylsulfon durch Umsetzung von Chlorbenzol mit sulfonierenden Mitteln erhalten hat.

6. Verfahren nach Anspruch 5, dadurch gekennzeichnet, daß man die nach Abtrennung des reinen 4,4'-Dichlordiphenylsulfons anfallenden Mutterlaugen nach destillativer Entfernung des Alkanols für eine erneute Herstellung von Dichlordiphenylsulfon heranzieht.

## Claims

1. A process for isolating 4,4'-dichlorodiphenyl sulfone from a dichlorodiphenyl sulfone mixture which, besides 4,4'-dichlorodiphenyl sulfone, contains from 0.5 to 20% by weight of 2,4'-dichlorodiphenyl sulfone and from 0.5 to 30% by weight of 3,4'dichlorodiphenyl sulfone, the percentages being based on 4,4'dichlorodiphenyl sulfone, which comprises treating this mixture with an alkanol at 20– 250°C in such a way that the mixture very largely dissolves, cooling the resulting mixture to precipitate 4,4'dichlorodiphenyl sulfone, and separating of the 4,4' -dichlorodiphenyl sulfone in a conventional manner.

2. A process as claimed in claim 1, wherein an alkanol of from 1 to 4 carbon atoms is used.

3. A process as claimed in claim 1, wherein the alkanol used is methanol.

4. A process as claimed in claim 1, wherein the amount of alkanol used is from 0.1 to 10 times the weight of 4,4'dichlorodiphenyl sulfone in the starting mixture.

5. A process as claimed in claim 1, wherein the dichlorodiphenyl sulfone mixture treated has been obtained in a conventional manner by reacting chlorobenzene with sulfonating agent.

6. A process as claimed in claim 5, wherein the mother liquor left behind after the pure 4,4'dichlorodiphenyl sulfone has been separated off and after the alkanol has been removed by distillation is reused for preparing dichlorodiphenyl sulfone.

## Revendications

1. Procédé d'isolement de la 4,4'-dichlorodiphénylsulfone à partir d'un mélange contenant des dichlorodiphénylsulfones et contenant par rapport à la 4,4'-dichlorodiphénylsulfone de 0,5 à 20% en poids de 2,4'-dichlorodiphénylsulfone et 0,5 à 30% en poids de 3,4'-dichlorodiphénylsulfone, caractérisé en ce qu'on traite ce mélange à des températures de 20° à 250°C avec un alcanol, de sorte qu'il se forme une solution la plus complète possible du mélange, on précipite la 4,4'-dichlorodiphénylsulfone par refroidissement du mélange ainsi obtenu et on sépare la 4,4'-dichlorodiphénylsulfone de façon connue.

2. Procédé selon la revendication 1, caractérisé en ce qu'on utilise un alcanol ayant de 1 à 4 atomes de carbone.

3. Procédé selon la revendication 1, caractérisé en ce qu'on utilise comme alcanol le méthanol.

4. Procédé selon la revendication 1, caractérisé en ce que la quantité d'alcool introduite s'élève à 0,1 à 10 fois la quantité en poids de 4,4'-dichlorodiphénylsulfone dans le mélange de départ.

5. Procédé selon la revendication 1, caractérisé en ce qu'on traite des mélanges de dichlorodiphénylsulfones qui ont été obtenus par des procédés connus par eux-mêmes pour la préparation de la 4,4'-dichlorodiphénylsulfone par réaction du chlorobenzène avec des agents de sulfonation.

6. Procédé selon la revendication 5, caractérisé en ce qu'on renvoie pour une nouvelle préparation de dichlorodiphénylsulfone, après élimination par distillation de l'alcool, la liqueur mère obtenue après séparation de la 4,4'-dichlorodiphénylsulfone pure.